(19) Europäisches Patentamt
European Patent Office
Office européen des brevets

(11) Veroffentlichungsnummer: **0 071 136**
**A2**

(12) # EUROPÄISCHE PATENTANMELDUNG

(21) Anmeldenummer: 82106473.0

(22) Anmeldetag: 19.07.82

(51) Int. Cl.³: **B 01 J 29/04**
B 01 J 29/28, C 10 G 3/00
C 01 B 33/28

(30) Priorität: 30.07.81 DE 3130033

(43) Veröffentlichungstag der Anmeldung: 09.02.83 Patentblatt 83/6

(84) Benannte Vertragsstaaten: BE DE FR GB IT NL SE

(71) Anmelder: BAYER AG
Zentralbereich Patente, Marken und Lizenzen
D-5090 Leverkusen 1, Bayerwerk(DE)

(72) Erfinder: Weisser, Jürgen, Dr.
Suedstrasse 15
D-4047 Dormagen 11(DE)

(72) Erfinder: Scharfe, Gerhard, Dr.
Berta-von-Suttner-Strasse 69
D-5090 Leverkusen 1(DE)

(72) Erfinder: Grolig, Johann, Dr.
Heinrich-Luebke-Strasse 22
D-5090 Leverkusen 1(DE)

(72) Erfinder: Waldmann, Helmut, Dr.
Henry-T.-von Boettinger-Strasse 15
D-5090 Leverkusen 1(DE)

(54) **Zinkhaltige Aluminiumsilikat-Katalysatoren, Verfahren zu ihrer Herstellung und ihre Verwendung.**

(57) Zinkhaltige Aluminiumsulikat-Katalysatoren, die erhältlich sind, indem man amorphes Aluminiumsilikat oder Gemische, aus denen ein solches Aluminiumsilikat erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternaren Ammoniumverbindungen, sowie mit in wäßrigen Alkalilösungen löslichen oder teilweise loslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert, sowie deren Herstellung und deren Verwendung zur Umwandlung von niederen Alkoholen und oder Ethern zu Kohlenwasserstoffen

EP 0 071 136 A2

Croydon Printing Company Ltd

BAYER AKTIENGESELLSCHAFT 5090 Leverkusen, Bayerwerk

Zentralbereich
Patente, Marken und Lizenzen Gai/bc/c

# Zinkhaltige Aluminiumsilikat-Katalysatoren, Verfahren zu ihrer Herstellung und ihre Verwendung

Die vorliegende Erfindung betrifft zinkhaltige Aluminiumsilikat-Katalysatoren, die aus amorphem Aluminiumsilikat oder Gemischen, aus denen amorphes Aluminiumsilikat erhältlich ist, erhältlich sind, ein Verfahren zur Herstellung solcher zinkhaltiger Aluminiumsilikat-Katalysatoren und deren Verwendung bei der Herstellung von Kohlenwasserstoffen aus aliphatischen Alkoholen und/oder Ethern.

Es ist bekannt, daß sich aliphatische organische Verbindungen und/oder deren Vorstufen an Aluminiumsilikat-Katalysatoren bei erhöhten Temperaturen in Kohlenwasserstoffgemische mit hohem Aromatanteil umwandeln lassen. Beispielsweise liefert die Umwandlung von Methanol an einem Zeolith-Katalysator vom Typ ZSM-5 neben Wasser ein Gemisch ein Gemisch aus Alkanen, Isoalkanen, Alkenen, Isoalkenen und überwiegend alkylsubstituierten Aromaten (s. beispielsweise US-PS 3 928 483).

Neben diesen gewünschten Kohlenwasserstoffen werden in geringerem Ausmaß stets Polymere und Koks gebildet. Dies hat zur Folge, daß die Aktivität dieser

Katalysatoren nach einer Laufzeit von einigen 100 Stunden merklich abnimmt, d.h. der Methanol-Umsatz sinkt auf Werte von unter 99 %, und es werden anstelle der gewünschten Kohlenwasserstoffe größere Mengen Dimethylether gebildet, wodurch die Raum/Zeit-Ausbeute an Kohlenwasserstoffen drastisch abfällt.

Es ist bekannt, daß man solche zeolithischen Katalysatoren durch Behandeln mit Luft oder Sauerstoff/Stickstoff-Gemischen bei sehr hohen Temperaturen unter Abbrennen des Kohlenstoffs und der Polymeren wieder regenerieren kann. So wird z.B. in Ind. Eng. Chem. Process Des. Dev., Vol. 17, No. 3, Seite 341 (1978) ein Verfahren zur Regeneration von durch Koksbildung geschädigten Katalysatoren beschrieben, bei dem Zeolith-Katalysatoren, die für die Umsetzung von Methanol zu Kohlenwasserstoffen bei Temperaturen von 370 bis 400°C eingesetzt waren, nach Abfall des Methanol-Umsatzes auf 99 bis 82 %, was einer Laufzeit von durchschnittlich 200 Stunden entsprach, mit Sauerstoff/Stickstoff-Gemischen regeneriert werden. Die Regeneration erfolgt nach einem Stickstoff-Purge bei Reaktionstemperatur zunächst mit 0,5 Vol.-% $O_2$ in Stickstoff bei 480 bis 540°C und dann, nach Steigerung des $O_2$-Gehaltes auf 20 Vol-%, durch 4-stündiges Erhitzen auf 600°C.

Nachteilig bei diesem Verfahren ist die Erhöhung der Temperatur um 200°C und mehr von der Reaktions- auf die Regenerationstemperatur, wodurch, insbesondere bei größeren technischen Anlagen, deren Katalysatormengen eine hohe Wärmekapazität besitzen, wertvolle

Zeit für das Aufheizen und Abkühlen des Katalysators bei der Regeneration aufgewendet werden muß. Dadurch verringert sich die Zeit während der der Katalysator für die gewünschte Reaktion zur Verfügung steht. Ein weiterer Nachteil ist, daß bei der periodischen Katalysatorregeneration bereits nach 7 Regenerationszyklen die Aktivität des Katalysators auf etwa 50 % der Anfangsaktivität abklingt (s. a.a.O. Seite 345-346).

Es wurde nun ein Katalysator gefunden, der diese Nachteile nicht aufweist.

Bei dem erfindungsgemäßen Katalysator handelt es sich um einen zinkhaltigen Aluminiumsilikat-Katalysator, der dadurch gekennzeichnet ist, daß er erhältlich ist, indem man amorphes Aluminiumsilikat mit einem Gehalt an Aluminiumoxid von 0,1 bis 20 Gew.-% oder Gemische, aus denen ein solches Aluminiumsilikat erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternären Ammoniumverbindungen, sowie mit in wäßrigen Alkalilösungen löslichen oder teilweise löslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert.

Die vorliegende Erfindung betrifft weiterhin ein Verfahren zur Herstellung von zinkhaltigen Aluminiumsilikat-Katalysatoren, das dadurch gekennzeichnet ist, daß man amorphes Aluminiumsilikat mit einem Gehalt an Aluminiumoxid von 0,1 bis 20 Gew.-% oder Gemische, aus denen ein solches Aluminiumsilikat erhältlich ist, mit aliphatischen $C_2$- bis $C_{24}$-Aminen und/oder cycloaliphatischen $C_2$- bis $C_{24}$-Aminen und/oder Ammoniumsalzen dieser Amine und/oder den aus diesen Aminen durch Quarternierung erhältlichen quarternären Aluminiumverbindungen, sowie mit in wäßrigen Alkalilösungen löslichen oder teilweise löslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert.

Ausgangsprodukte für die Herstellung der erfindungsgemäßen Katalysatoren können entweder amorphe Aluminiumsilikate mit einem Gehalt an Aluminiumoxid von 0,1 bis 20 Gew.-% sein oder Gemische, aus denen solche Aluminiumsilikate erhältlich sind. Vorzugsweise setzt man ein amorphes Aluminiumsilikat ein mit einem Gehalt an Aluminiumoxid von 0,5 bis 10 Gew.-% oder Gemische, aus denen ein solches Aluminiumsilikat erhältlich ist; besonders bevorzugt ein amorphes Aluminiumsilikat mit einem Gehalt an Aluminiumoxid von 1 bis 5 Gew.-% oder Gemische, aus denen ein solches Aluminiumsilikat erhältlich ist.

Gemische, aus denen amorphe Aluminiumsilikate mit den vorstehend angegebenen Gehalten an Aluminiumoxid erhältlich sind, können die verschiedensten Gemische

sein, die eine silikatische oder Siliciumdioxid enthaltende Komponente und eine, als Quelle für Aluminiumoxid und/oder -hydroxid geeignete Aluminiumverbindung im entsprechenden Verhältnis enthalten, wobei beide Komponenten unabhängig voneinander in gelöster, suspendierter oder fester Form vorliegen können.

Ein bevorzugtes Gemisch dieser Art enthält amorphes Siliciumdioxid mit einem Gehalt an Siliciumdioxid von über 90 Gew.-% und eine oder mehrere lösliche, als Quelle für Aluminiumoxid und/oder Aluminiumhydroxid geeignete Verbindungen des Aluminiums, entsprechend einem Gehalt von 0,1 bis 20, vorzugsweise 0,5 bis 10, besonders bevorzugt 1 bis 5 Gew.-% Aluminiumoxid, bezogen auf die Summe der Gewichte von Siliciumdioxid und Aluminiumoxid.

Als Quelle für Aluminiumoxid und/oder -hydroxid sind z.B. alle Aluminiumverbindungen geeignet, die in wäßriger Lösung der Hydrolyse unterliegen und in einem Gleichgewicht mit Verbindungen stehen, in denen sich mindestens eine Hydroxygruppe am Aluminiumatom befindet. Es lassen sich aber auch in Wasser oder organischen Lösungsmitteln neutral reagierende Aluminiumverbindungen verwenden.

Geeignet sind beispielsweise die verschiedensten Aluminiumsalze und/oder Aluminiumkomplexverbindungen. Beispielsweise seien genannt: Aluminiumchlorid, -nitrat, -sulfat, -acetat, -acetylacetonat, -oxidhydrat, -fluorid, -bromid, -oxalat, Aluminiumsalze von $C_1$- $C_{20}$-

Carbonsäuren, sowie Kaliumaluminiumsulfat, Natriumaluminiumsulfat, Aluminiumalkoholate und Alkalialuminate. Vorzugsweise verwendet man Aluminiumoxidhydrat, Aluminiumnitrat, Natriumaluminat, Aluminiumacetat und/oder Aluminiumchlorid. Die Umsetzung von amorphem Siliciumdioxid mit Verbindungen des Aluminiums zu Aluminiumsilikat kann gleichzeitig mit der Behandlung mit Zinkverbindungen oder in einem vor- oder nachgeschalteten Reaktionsschritt erfolgen.

Beispielsweise kann diese Umsetzung so vorgenommen werden, daß man das amorphe Siliciumdioxid mit der Lösung einer Aluminiumverbindung in Wasser oder einem polaren organischen Lösungsmittel tränkt und anschließend einer Wärmebehandlung unterwirft. Als polare organische Lösungsmittel können beispielsweise Alkohole, Ketone, Ether, Ester, Nitrile, Amide und/oder Sulfoxide verwendet werden. Vorzugsweise wird eine wäßrige Lösung einer Aluminiumverbindung verwendet.

In bestimmten Fällen kann es erforderlich oder vorteilhaft sein, dem Gemisch aus Aluminiumverbindung und Wasser oder Lösungsmittel Zusätze zuzufügen, um eine, gegebenenfalls schnellere, Auflösung der Aluminiumverbindung zu erreichen. So ist es z.B. beim Einsatz von Aluminiumoxidhydrat vorteilhaft, eine starke Base, z.B. Natronlauge, zuzusetzen, wodurch das im allgemeinen schwer lösliche Aluminiumoxidhydrat in ein wesentlich besser lösliches Aluminat überführt wird.

Die Wärmebehandlung kann z.B. so vorgenommen werden, daß man das mit der Lösung einer Aluminiumverbindung getränkte amorphe Siliciumdioxid langsam so weit erwärmt, daß das Lösungsmittel, z.B. Wasser, verdampft und die vollständig oder teilweise hydrolysierte Aluminiumverbindung in dem Kieselsäurematerial verbleibt. Selbstverständlich kann das Lösungsmittel auch durch Evakuieren oder einfaches Trocknen an der Luft entfernt werden. Weiterhin kann das auf diese Weise mit Verbindungen des Aluminiums umgesetzte Siliciumdioxid einem Calcinierungsprozeß bei Temperaturen von beispielsweise 600 bis 1200°C unterworfen werden.

Wenn amorphes Aluminiumsilikat oder amorphes Siliciumdioxid eingesetzt wird, werden diese vorzugsweise als Formkörper eingesetzt. Besonders bevorzugt weisen solche Formkörper einen minimalen Durchmesser von über 1 mm und einen maximalen Durchmesser von unter 20 mm auf. Solche Formkörper sind im Handel erhältlich. Es kann vorteilhaft sein, zur Herstellung der erfindungsgemäßen zinkhaltigen Aluminiumsilikat-Katalysatoren vorgesehenes amorphes Aluminiumsilikat oder Siliciumdioxid, insbesondere wenn diese als Formkörper vorliegen, vor der Behandlung mit Aminen, Ammoniumsalzen und/oder quartärnären Ammoniumverbindungen, sowie vor der Behandlung mit Zinkverbindungen und im Falle von Siliciumdioxid vor der Umsetzung mit Aluminiumverbindungen, in einen Zustand erhöhter Reaktionsbereitschaft zu bringen. Dies kann beispielsweise erfolgen, indem man die Formkörper glüht und gegebenenfalls anschließend entgast. Das Glühen kann beispielsweise

bei Temperaturen im Bereich 600 bis 1100 °C für beispielsweise 1 bis 6 Stunden erfolgen. Die Entgasung kann beispielsweise erfolgen, indem man die Formkörper nach dem Glühen in ein evakuierbares Gefäß einbringt und dieses evakuiert.

Weitere Gemische, aus denen amorphes Aluminiumsilikat mit einem Gehalt an Aluminiumoxid von 0,1 bis 20, 0,5 bis 10 oder 1 bis 5 Gew.-% erhältlich ist, sind solche, die lösliche oder suspendierte Ausgangskomponenten in entsprechenden Verhältnissen enthalten und wobei die Ausgangskomponenten in wäßrigem Medium solche amorphen Aluminiumsilikate bilden können. Beispiele für solche Ausgangskomponenten sind Alkalimetallsilikate (sog. "Wassergläser"), kolloidal gelöste Kieselsäure (sog. "Kieselsol"), pyrogene Kieselsäure, Ester aus Kieselsäure und aliphatischen Alkoholen (beispielsweise Tetramethoxy- und Tetraethoxysilikat), Siliciumtetrahalogenide (beispielsweise Siliciumtetrachlorid), sowie diejenigen Verbindungen des Aluminiums, die vorstehend als geeignete Quelle für Aluminiumoxid und/oder Aluminiumhydroxid angeführt wurden.

Aus derartigen Ausgangskomponenten sind amorphe Aluminiumsilikate erhältlich, indem man beispielsweise eine Lösung und/oder Suspension einer Silicium-$4^+$ enthaltenden Verbindung, vorzugsweise in wässrigem, alkalisch reagierendem Medium mit einer in Wasser oder polaren organischen Lösungsmitteln gelösten oder teilweise gelösten Verbindung des Aluminiums zur Reaktion bringt.

Stellvertretend für solche Umsetzungen seien hier die Reaktionen von a) Natriumsilikat mit Natriumaluminat, b) Natriumaluminat mit Kieselsol und c) Tetraethoxysilikat mit Aluminiumisopropylat genannt.

Die erfindungsgemäße Behandlung mit Aminen, Ammoniumsalzen und/oder quarternären Ammoniumverbindungen wird vorzugsweise mit einer oder mehreren Verbindungen aus einer oder mehreren der folgenden Gruppen vorgenommen:

Monoamine der Formel

$$N(R_1)(R_2)(R_3) \qquad (I)$$

in der

$R_1$, $R_2$ und $R_3$ gleich oder verschieden sein können und für $C_2$-$C_6$-Alkyl, $C_2$-$C_6$-Hydroxyalkyl oder $C_2$-$C_6$-Aminoalkyl stehen und in der $R_1$ und/oder $R_2$ auch für Wasserstoff steht.

Diamine der Formel

$$(R_4)_2N-[C(R_5)_2]_n-N(R_6)_2 \qquad (II)$$

in der

$R_4$, $R_5$ und $R_6$ gleich oder verschieden sein können und für $C_1$-$C_2$-Alkyl, $C_1$-$C_2$-Hydroxyalkyl, $C_1$-$C_2$-Aminoalkyl oder Wasserstoff stehen und in der n für eine ganze Zahl von 2 bis 6 steht.

Monoammoniumsalze der Formel

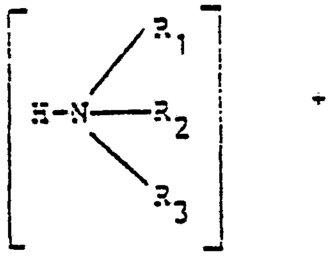

$X^-$ (III)

in der

$R_1$, $R_2$ und $R_3$ die bei Formel (I) angegebene Bedeutung haben und in der X für Cl, Br, J, $HSO_4$, $HCO_3$, $H_2PO_4$ oder $BF_4$ steht.

Monoammoniumsalze der Formel

$$[H(R_4)_2N-[C(R_5)_2]_n-N(R_6)_2]^+ \quad X^- \qquad (IV)$$

in der

$R_4$, $R_5$, $R_6$ und n die bei Formel (II) angegebene Bedeutung haben und in der X die bei Formel (III) angegebene Bedeutung hat.

Quarternäre Ammoniumverbindungen der Formel

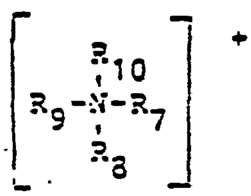

$X^-$ (V)

in der

$R_7$, $R_8$, $R_9$ und $R_{10}$ gleich oder verschieden sein können und für $C_2$-$C_4$-Alkyl, $C_2$-$C_4$-Hydroxyalkyl oder $C_2$-$C_4$-Aminoalkyl stehen, oder

in der

zwei der Reste $R_7$, $R_8$, $R_9$ und $R_{10}$ für $C_1$-$C_4$-Alkyl, $C_2$-$C_4$-Hydroxyalkyl oder $C_2$-$C_4$-Aminoalkyl und die beiden anderen Reste für $C_2$-$C_{12}$-Alkyl, Benzyl, $C_2$-$C_{12}$-Hydroxyalkyl, $C_2$-$C_{12}$-Aminoalkyl oder $C_4$-$C_8$-Cycloalkyl stehen und

X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

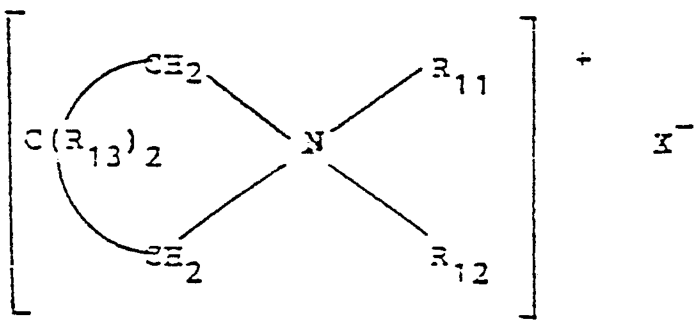

(VI)

in der

$R_{11}$ und $R_{12}$ gleich oder verschieden sein können und für $C_1$-$C_8$-Alkyl, $C_2$-$C_8$-Hydroxyalkyl oder $C_2$-$C_8$-Aminoalkyl stehen,

$R_{13}$ für Wasserstoff oder $C_1$-$C_8$-Alkyl steht, und

X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

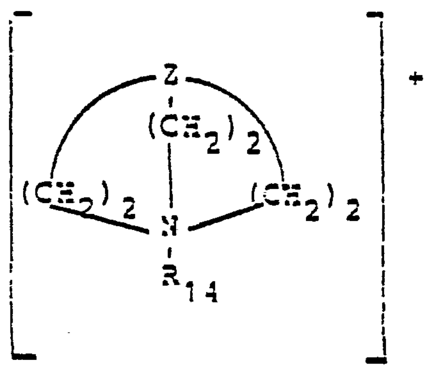

$X^-$ (VII)

in der

$R_{14}$ für $C_1$-$C_8$-Alkyl oder 2-Hydroxyethyl steht,

Z für Stickstoff, $-CR_{15}$ oder $-NR_{16}^+$ $X^-$ steht, wobei $R_{15}$ für $C_1$-$C_8$-Alkyl steht und $R_{16}$ die gleiche Bedeutung hat, wie vorstehend für $R_{14}$ angegeben und

X OH ist oder die bei Formel (III) angegebene Bedeutung hat,

wobei quarternäre Ammoniumverbindungen mit insgesamt mehr als 24 C-Atomen ausgeschlossen sind.

Innerhalb der Verbindungen gemäß den Formeln (I) bis (VII) sind solche bevorzugt, die insgesamt 2 bis 16 C-Atome enthalten.

Besonders bevorzugt werden als Amine, Ammoniumsalze und/oder quarternäre Ammoniumverbindungen eine oder mehrere der folgenden Verbindungen eingesetzt:

Tetrapropylammoniumhydroxid, -chlorid, -bromid, -jodid, Tetrabutylammoniumhydroxid, -chlorid, -bromid, -jodid, Tris-(2-hydroxyethyl)-n-propylammoniumhydroxid, -chlorid, -bromid, -jodid, sowie Tetramethylendiamin, Pentamethylendiamin, Hexamethylendiamin, n-Propylamin, n-Butylamin, Ethanolamin, Diethanolamin, Triethanolamin, wobei die genannten Amine in freier Form oder an einem Stickstoffatom protoniert und mit Chlorid, Bromid, Jodid, Hydrogensulfat, Dihydrogenphosphat, Hydrogencarbonat und/oder Tetrafluoroborat als Anion eingesetzt werden können.

Ganz besonders bevorzugt wird Tetrapropylammoniumbromid, Tetrapropylammoniumhydroxid, Tetrabutylammoniumbromid, Tetrabutylammoniumhydroxid und Tris-(2-hydroxyethyl)-n-propylammoniumhydroxid eingesetzt.

Die Menge, in der erfindungsgemäß Amine, Ammoniumsalze und/oder quarternäre Ammoniumverbindungen (im folgenden gemeinsam als Stickstoffverbindungen bezeichnet) eingesetzt werden können, kann in einem weiten Bereich variiert werden. Beispielsweise kann man die Stickstoffverbindungen in einer Menge von 5 bis 100 Gew.-%, bezogen auf das Gewicht des im eingesetzten Ausgangsmaterial enthaltenen Siliciumdioxids plus Aluminiumoxids einsetzen. Vorzugsweise setzt man 10 bis 50 Gew.-% einer oder mehrerer Verbindungen aus der Gruppe der zuvor beschriebenen Stickstoffverbindungen ein, bezogen auf das Gewicht des im eingesetzten Ausgangsmaterial enthaltenen Siliciumdioxids plus Aluminiumoxids.

Die Menge Wasser, die erfindungsgemäß einzusetzen ist, kann ebenfalls in einem weiten Bereich variiert werden.

Es ist vorteilhaft, mindestens soviel Wasser einzusetzen, daß das Gemisch aus Wasser und Stickstoffverbindung(en) ausreicht, um das eingesetzte Ausgangsmaterial vollständig zu durchtränken. Selbstverständlich können auch größere Mengen an Wasser eingesetzt werden, beispielsweise so viel, daß das eingesetzte Ausgangsmaterial völlig bedeckt wird oder eine größere Menge.

Für das erfindungsgemäße Verfahren ist es wesentlich, daß das Gemisch aus Stickstoffverbindung(en) und Wasser einen pH-Wert von größer als 8 aufweist. Vorzugsweise beträgt der pH-Wert dieses Gemisches mindestens 9, besonders bevorzugt mindestens 10.

Zur Erreichung von höheren pH-Werten kann es erforderlich sein, dem Gemisch aus Stickstoffverbindung(en) und Wasser alkalisch reagierende Stoffe zuzusetzen. Außer stark alkalisch reagierenden Verbindungen aus der Gruppe der zuvor beschriebenen Stickstoffverbindungen kommen hierfür auch die verschiedensten organischen und/oder anorganischen Verbindungen mit ausreichender Alkalität in Frage. Beispiele für derartige organische und anorganische Verbindungen sind Amine, wie Methyl-, Dimethyl-, Trimethyl-, Ethyl-, Diethyl-, Triethyl-, Methylethyl-, Propyl- und Butylamin, Anilin, Methyl-, Ethyl-, Dimethyl-, Diethyl-, Methylethylanilin, Pyrrol, Imidazol, Pyrazol, Pyridin, Pyrazin, Pyrimidin, Pyridazin, Indol, Purin, Chinolin, Pyrrolidin, Piperidin, Piperazin; organische Hydroxide wie Tetramethylammoniumhydroxid, Cholin und dessen Derivate; anorganische

Oxide und Hydroxide, wie Ammoniakwasser, Alkali- und Erdalkalioxide und -hydroxide, insbesondere Natrium-, Kalium-, Lithium-, Magnesium-, Calcium- und Bariumoxid oder -hydroxid; Salze aus starken Basen und schwachen Säuren, wie Carbonate, Phosphate, Silikate, Acetate, Fluoride, Stannite, Stannate, Borate, Wolframate, Molybdate, Sulfide, Sulfite von Natrium, Kalium, Lithium, Magnesium, Calcium und Barium. Vorzugsweise kommen als Zusätze solche in Frage, die in Wasser gut löslich sind und mit den Stickstoffverbindungen, den Zinkverbindungen und den sonstigen eingesetzten Ausgangsmaterialien nicht in unerwünschter Weise reagieren.

Auch aus anderen Gründen kann es vorteilhaft sein, dem Gemisch aus Stickstoffverbindung(en) und Wasser Zusätze zuzufügen. So wurde beispielsweise festgestellt, daß bei Zusätzen von Alkalicarbonaten, Alkaliphosphaten, Alkalifluoriden, Alkalisulfiden, Alkalicarboxylaten, Alkalisilikaten und/oder Alkaliwolframaten geformte Katalysatoren auf der Basis kristalliner Aluminiumsilikate erhalten werden können, die eine besonders gute Bruchhärte aufweisen. Dies ist insbesondere bei Zusätzen von Alkaliphosphaten, Alkalicarbonaten, Alkalisilikaten, Alkalisulfiden und/oder Alkaliwolframaten der Fall.

Die Zusätze zur Erhöhung des pH-Wertes und/oder zur Erzielung guter Bruchhärten können in weiten Grenzen variiert werden. Im allgemeinen sind Zusätze in einer Menge ausreichend, die bezogen auf das eingesetzte oder sich bildende Aluminiumsilikat formal und ohne Berücksichtigung von Dissoziationsgleichgewichten 0,01 bis 10, vorzugsweise 0,1 bis 5 Gew.-%, Hydroxidionen freisetzen können.

Bei den erfindungsgemäß einzusetzenden Verbindungen des Zinks kommen solche in Frage, die bei pH-Werten von über 8 in wäßrigem Medium löslich oder zumindest teilweise löslich sind. Beispielsweise sind Zinkoxid, Zinkhydroxid, Zinkate, Zinksalze und/oder komplexe Zinkverbindungen geeignet.

Es kommen beispielsweise Zinkate, Zinkat-Derivate und/oder Diammin- oder Tetramminzinksalze in Frage, wie Mononatriumzinkat, Dinatriumzinkat, Natriumorthozinkat, Natriumcyanozinkat, Monokaliumzinkat, Dikaliumzinkat, Kaliumorthozinkat, Kaliumcyanozinkat, Hydroxoderivate der vorstehend genannten Natrium- und Kaliumzinkate, Diamminzinkacetat, -bromid, -chlorid, -hydroxid, -jodid, -nitrat, -oxalat, -sulfat, -tartrat, Tetramminzinkacetat, -bromid, -chlorid, -hydroxid, -jodid, -nitrat, -oxalat, -sulfat, -tartrat.

Ebenso geeignet sind Komplexverbindungen des Zinks, beispielsweise solche, die aus Zinkacetat, -bromid, -chlorid, -fluorid, -hydroxid, -jodid, -nitrat, -oxalat, -oxid, -sulfat, -sulfid, -sulfit und/oder -tartrat sowie über Sauerstoff und/oder Stickstoff koordinierenden Liganden entstehen können.

Geeignete Liganden sind außer Ammoniak beispielsweise Hydrazin, Hydroxylamin, Mono-, Di- und Trimethylamin, Mono-, Di- und Triethylamin, Ethylendiamin, Diethylentriamin, Hexamethylendiamin, Phenylhydrazin, Anilin, Toluidin,

Xylidin, Pyridin, Dipyridin, Nitrilotriessigsäure, Ethylendiamintetraessigsäure, Mono-, Di- und Triethanolamin, Aminoessigsäure, Harnstoff, Thioharnstoff, Acetylaceton, Glycerin und Brenzcatechin.

Für die erfindungsgemäßen Katalysatoren bzw. für ihre Herstellung ist es unwesentlich, ob die vorstehend genannten Zinkverbindungen separat hergestellt oder in situ im basischen Reaktionsmedium durch einfaches Hinzufügen von Zinkverbindungen und gegebenenfalls einem komplexierenden Mittel erzeugt werden.

Als besonderer Vorteil ist anzusehen, daß man auch Zinkoxid und/oder Zinkhydroxid dem Reaktionsmedium zusetzen kann, da sich diese Verbindungen im basischen Medium zu partiell löslichen Zinkaten umsetzen. Dies gilt selbstverständlich auch für Zinkverbindungen, die in wäßriger Lösung in Gegenwart von Alkali Zinkhydroxid liefern und mit weiterem Alkali zu Zinkaten reagieren können. Die dabei u.U. entstehenden Nebenprodukte (z.B. Salze aus Alkalimetallkationen und dem Anion der eingesetzten Zinkverbindung) stören das erfindungsgemäße Verfahren nicht. Sie üben im Gegenteil eine mineralisierende, d.h. kristallisationsfördernde Wirkung auf das amorphe Aluminiumsilikat aus.

Als Zinkverbindungen, die im alkalischen Medium über Zinkhydroxid zu Zinkaten bzw. Hydroxokomplexen reagieren können, sind beispielsweise geeignet Zinkacetat, Zinkbromid, Zinkchlorid, Zinkcyanid, Zinkfluorid, Zinkfluorborat, Zinkferrocyanid, Zinkformiat, Zink-

gallat, Zinkhydroxid, Zinkjodid, Zinkjodat, Zinknitrat, Zinkoxalat, Zinkoxid, Zinkphosphat, Zinkrhodanid, Zinksulfat, Zinksulfid, Zinksulfit und Zinktartrat.

Die Verbindungen des Zinks können z.B. in einer Menge entsprechend 0,1 bis 50 Gew.-% Zinkoxid, bezogen auf das im Reaktionsgemisch vorhandene oder entstehende Aluminiumsilikat, eingesetzt werden.

Bevorzugt entspricht diese Menge 0,5 bis 10 Gew.-% Zinkoxid.

Die Stickstoffverbindung(en), das Wasser, die einzusetzenden Ausgangsmaterialien, die Verbindungen des Zinks, sowie die gegebenenfalls einzusetzenden Zusätze können in beliebiger Reihenfolge zusammengegeben werden.

Für die Herstellung erfindungsgemäßer Katalysatoren kann man beispielsweise eine separate Behandlung (Tränkung, Imprägnierung und/oder Umsetzung) des Ausgangsmaterials mit Verbindungen des Zinks, gefolgt von der Behandlung mit Stickstoffverbindung(en), gegebenenfalls Zusätzen und wäßriger, alkalisch reagierender Lösung durchführen.

Mit gleichem Erfolg können die Zinkverbindungen dem wäßrigen Reaktionsgemisch, das bereits alle übrigen zur Umwandlung in kristallines Aluminiumsilikat erforderlichen Reaktionskomponenten enthält, zugesetzt werden.

Die Ausgangsmaterialien lassen sich in einfacher Weise in zinkhaltige, kristalline Aluminiumsilikat-Katalysatoren umwandeln, wenn man sie in einem wäßrigen Milieu bei einem pH-Wert von größer als 8, zusammen mit unter diesen Bedingungen löslichen oder zumindest teilweise löslichen Verbindungen des Zinks sowie Aminen, Ammoniumsalzen und/oder quartären Ammoniumverbindungen Temperaturen im Bereich 50 bis 250°C aussetzt, anschließend auswäscht, trocknet und bei 300 bis 600°C calciniert.

Selbstverständlich kann man auch das alkalisch-wäßrige und Zink- und Stickstoffverbindung(en) enthaltende Milieu mit einem Zusatz von löslichen, als Quelle für Aluminiumoxid und/oder Aluminiumhydroxid geeigneten Verbindungen des Aluminiums versehen, sofern amorphes Siliciumdioxid als Ausgangsmaterial verwendet wird. Durch diese Maßnahme kann auf eine separate Umsetzung des amorphen Siliciumdioxids mit Verbindungen des Aluminiums verzichtet werden.

Hinsichtlich der Qualität des angestrebten zinkhaltigen kristallinen Aluminiumsilikats ist es unerheblich, ob von einem amorphem und in irgendeiner Form separat mit Verbindungen des Aluminiums umgesetztem Siliciumdioxid oder direkt von einem amorphem Aluminiumsilikat oder von amorpher Kieselsäure bzw. von Kieselsäure liefernden Verbindungen des Siliciums und Verbindungen des Aluminiums enthaltenden Reaktionsgemischen ausgegangen wird.

wenn die Behandlung mit Aminen, Ammoniumsalzen und/oder quaternären Ammoniumverbindungen gleichzeitig mit der Behandlung mit Zink- und gegebenenfalls Aluminiumverbindungen erfolgt, so kann es erforderlich sein, daß größere Mengen einer basisch reagierenden Verbindung zugesetzt werden müssen, um den für diese Behandlung notwendigen pH-Wert von größer als 8 zu erreichen. Dies ist insbesondere dann erforderlich, wenn Zink und gegebenenfalls Aluminiumverbindungen verwendet werden, die in Wasser durch Hydrolyse stark sauer reagieren, wie beispielsweise Zinkchlorid und Aluminiumchlorid. Als basisch reagierende Verbindungen kommen beispielsweise eine oder mehrere der vorstehend beschriebenen Stickstoffverbindungen sowie der anorganischen und in Wasser alkalisch reagierenden Stoffe in Frage.

Die Stickstoffverbindung(en), die vorstehend beschriebenen Verbindungen des Zinks, das Wasser und das einzusetzende Ausgangsmaterial bzw. dessen Vorstufen, sowie die gegebenenfalls einzusetzenden Zusätze können in beliebiger Reihenfolge zusammengegeben werden. Im allgemeinen geht man so vor, daß man aus Wasser, Stickstoffverbindung(en), Verbindung(en) des Zinks und gegebenenfalls einem oder mehreren Zusätzen eine Lösung oder Suspension herstellt und das einzusetzende Ausgangsmaterial damit übergießt oder ein aus reaktiven $SiO_2$- und $Al_2O_3$-Vorstufen hergestelltes Hydrogel damit versetzt.

Nach dem Zusammengeben von Wasser, Stickstoffverbindung(en), Verbindung(en) des Zinks, dem einzusetzenden Ausgangsmaterial und gegebenenfalls Zusätzen wird das so gebildete Reaktionsgemisch bei Temperaturen im

0071136



Bereich von 50 bis 250°C, vorzugsweise im Bereich von 80 bis 180°C, ausgesetzt. Dabei wird das amorphe Ausgangsmaterial, gegebenenfalls unter Beibehaltung seiner äußeren Form, zum größten Teil oder vollständig in kristallines zinkhaltiges Aluminiumsilikat überführt. Die Umwandlungsgeschwindigkeit hängt im wesentlichen von der Beschaffenheit des Ausgangsmaterials und der Temperatur ab. Man kann z.B. durch Debye-Scherrer (Röntgenbeugungs-)Analyse von Proben den jeweils erreichten Kristallinitätsgrad feststellen. Mit steigender Reaktionstemperatur nimmt die notwendige Behandlungszeit stark ab. Im allgemeinen können gute Ergebnisse erhalten werden, wenn man das Reaktionsgemisch beispielsweise 20 bis 60 Stunden bei ca. 180°C oder etwa 80 bis 150 Stunden bei ca. 120°C oder etwa 350 bis 500 Stunden bei ca. 80°C beläßt. In einzelnen Fällen können jedoch auch von diesen Richtwerten mehr oder weniger stark abweichende Behandlungszeiten notwendig sein.

Wenn die Behandlung bei Temperaturen vorgenommen werden soll, bei der Teile des Reaktionsgemisches unter Normaldruck flüchtig sind, so wird zweckmäßigerweise in einem druckbeständigen, verschließbaren Gefäß gearbeitet. Es ist nicht notwendig, während der Behandlung zu rühren.

Wenn durch die Behandlung der gewünschte Kristallinitätsgrad erreicht worden ist, so wird das feste Reaktionsprodukt zunächst von der gegebenenfalls vorhandenen überschüssigen wäßrigen Phase abgetrennt, bei-

spielsweise durch eine mechanische Abtrennung wie Dekantieren oder Filtrieren. Die abgetrennte wäßrige Phase kann, gegebenenfalls nach Ergänzung der verbrauchten Bestandteile, wiederverwendet werden. Es ist dann, auch wenn nach der Behandlung keine mechanisch abtrennbare wäßrige Phase vorhanden ist, noch erforderlich, das feste Reaktionsprodukt bei 300 bis 600°C, vorzugsweise bei 400 bis 600°C, zu calcinieren. Diese Calcinierung wird durchgeführt, nachdem das feste Reaktionsprodukt z.B. mit Wasser neutral gewaschen und getrocknet worden ist.

Man erhält so, zum größten Teil oder vollständig kristallines, zinkhaltiges Aluminiumsilikat, das - sofern Formkörper als Ausgangsmaterial verwendet werden - die angenäherten Dimensionen des Ausgangsmaterials aufweist. Das kristalline Produkt besitzt meistens schon eine gewisse katalytische Aktivität. In vielen Fällen kann die katalytische Aktivität noch verbessert werden, beispielsweise indem man das Reaktionsprodukt des erfindungsgemäßen Verfahrens in an sich bekannter Weise einem Ionenaustausch mit Ammoniumionen oder Wasserstoffionen unterzieht und es anschließend nochmals calciniert.

Es kann auch vorteilhaft sein, insbesondere um hochaktive Katalysatoren für spezielle Zwecke zu erhalten, während oder nach dem Ionenaustausch weitere, die katalytischen Eigenschaften verbessernde Komponenten in das zinkhaltige Aluminiumsilikat einzubringen. Als derartige Komponenten kommen beispielsweise Schwerme-

talle, Edelmetalle, Nichtmetallverbindungen und/oder Verbindungen der Seltenen Erden in Frage, die nach an sich bekannten Techniken eingebracht werden können.

Die erfindungsgemäß hergestellten, zinkhaltigen, kristallinen Aluminiumsilikate eignen sich als Katalysatoren für die Umwandlung niederer aliphatischer Alkohole und/oder Ether, speziell Methanol und/oder Dimethylether in Kohlenwasserstoffe.

Bei der Umwandlung von Methanol und/oder Dimethylether in Kohlenwasserstoffe, die im allgemeinen im Temperaturbereich von 300 bis 400°C durchgeführt wird, entstehen neben den gewünschten Reaktionsprodukten auch geringe Mengen Polymere und Koks, die im Verlauf der katalytischen Umwandlung zu einer Katalysator-Desaktivierung beitragen. Katalysatoren, die nach dem gegenwärtigen Stand der Technik hergestellt werden, müssen deshalb jeweils nach einigen hundert Stunden bei Temperaturen bis 600°C regeneriert werden.

Die erfindungsgemäßen Katalysatoren zeichnen sich durch eine ungewöhnliche, nicht vorhersehbare und bisher nicht bekannte Eigenschaft aus, sie lassen sich nämlich bereits in einem Temperaturbereich von 300 bis 400°C, d.h. bei der technisch üblichen Reaktionstemperatur der katalytischen Methanol- bzw. Dimethyletherumwandlung, in einfacher Weise durch Überleiten von Wasserdampf und/oder Sauerstoff enthaltenden Gasen regenerieren, wobei auch nach mehrmaligen Regenerationen die ursprüngliche Aktivität des Katalysators wiederhergestellt werden kann.

Diese Regeneration kann beispielsweise durch Wasserdampf allein, durch Wasserdampf/Sauerstoff- bzw. Wasserdampf/Sauerstoff/Stickstoff-Gemische oder durch Sauerstoff/Stickstoff-Gemische (z.B. Luft) erfolgen.

Die Mengen des anzuwendenden Wasserdampfes können in weiten Grenzen variiert werden, wobei nach oben eine Begrenzung durch die Wirtschaftlichkeit und nach unten durch die Stöchiometrie der Wassergasreaktion gegeben ist. In ähnlicher Weise kann die Menge des Sauerstoffs begrenzt werden, sofern sauerstoffhaltige Gasgemische eingesetzt werden. Die Konzentrationen des Sauerstoffs in den eingesetzten Gasgemischen kann beispielsweise im Bereich von 0,1 bis 20 Vol.-%, vorzugsweise im Bereich von 0,5 bis 10 Vol.-%, bezogen auf das Regenerationsgas, betragen.

Es ist offenkundig, daß besonders die Regeneration mit Wasserdampf oder überwiegend aus Wasserdampf bestehenden Gasmischungen bei Temperaturen von 300 bis 400°C bzw. bei Temperaturen, die der Reaktionstemperatur entsprechen, beträchtliche ökonomische und verfahrenstechnische Vorteile bietet. So entfallen bei den erfindungsgemäßen Katalysatoren die Zeiten, die bei bisher üblichen Katalysatoren zunächst für die Aufheizung auf Regenerationstemperatur und danach wieder für die Abkühlung auf Reaktionstemperatur erforderlich waren, d.h. die Zeit, in der der Katalysator für die gewünschte Reaktion zur Verfügung steht, ist beträchtlich länger als bei üblichen Katalysatoren.

Die erfindungsgemäßen Katalysatoren zeichnen sich

weiterhin durch eine extrem hohe Resistenz gegenüber dem sogenannten "steaming" aus. Hierunter versteht man insbesondere die Reaktion konventioneller Aluminiumsilikat-Katalysatoren mit Wasserdampf, die zu strukturellen Veränderungen des Aluminosilikat-Kristallgitters und damit zu einer irreversiblen, nicht auf Ablagerungen beruhenden Katalysator-Desaktivierung führt.

Die erfindungsgemäßen Katalysatoren, die in der vorstehend beschriebenen Weise regeneriert werden, behalten auch nach vielen Regenerationsschritten ihre ursprüngliche Aktivität.

Die besonderen Vorteile der vorliegenden Erfindung lassen sich beispielsweise anhand der folgenden Verfahrensweise erkennen:

Werden die erfindungsgemäßen Katalysatoren z.B. bei der katalytischen Methanolumwandlung eingesetzt, so entsteht als Reaktionsprodukt im allgemeinen ein aromatenreiches Benzin neben einer olefinreichen Gasfraktion. Letztere kann vollständig oder teilweise in die Reaktionsstufe zurückgeführt werden, um die Benzinausbeute zu erhöhen. Wird bei dieser sogenannten Kreisgasfahrweise nach einer Betriebsdauer von mehreren hundert Stunden die Methanolzufuhr abgestellt und leitet man statt dessen für einige Stunden Wasserdampf oder Wasserdampf-Luft-Gemische über den Katalysator, so läßt sich von vornherein die Bildung von schädlichen Ablagerungen unterdrücken oder sogar ganz verhindern. Die erfindungsgemäßen Katalysatoren behalten ihre volle Aktivität, sofern sie zwischenzeitlich wie vorstehend beschrieben regeneriert werden, über wesentlich längere Zeiträume als

bisherige Katalysatoren, beispielsweise für mehrere tausend Stunden.

Selbstverständlich ist diese Behandlung mit Wasserdampf bzw. Wasserdampf-Luft-Gemischen nicht an die Kreisgasfahrweise der Methanolumwandlung gebunden; sie läßt sich mit gleichem Erfolg auch bei einer Fahrweise im geraden Durchgang anwenden.

Bei einer beispielhaften, in technischem Maßstab durchführbaren Ausführungsform der Erfindung kann wie folgt verfahren werden:

Amorphes Siliciumdioxid mit einem Siliciumdioxidgehalt von über 90 Gew.-% wird bei einer Temperatur von über 800°C geglüht und danach im Vakuum entgast. Das so vorbehandelte Ausgangsmaterial wird mit einem Gemisch aus wäßriger Natronlauge, gelöster Aluminiumverbindung, gelöster oder suspendierter Zinkverbindung und einer oder mehreren Stickstoffverbindungen der beschriebenen Art versetzt, wobei die Menge dieses Gemisches einem Vielfachen des Porenvolumens des Ausgangsmaterials entspricht. Die Natronlauge kann beispielsweise 0,1 bis 5 Gew.-% Hydroxidionen (bezogen auf eingesetztes oder sich bildendes Aluminiumsilikat) enthalten, gegebenenfalls auch mehr. Als Aluminiumverbindungen werden vorzugsweise Natriumaluminat, Aluminiumsulfat, Aluminiumchlorid, Natriumaluminiumsulfat, Kaliumaluminiumsulfat, Aluminiumacetat oder Aluminiumfluorid in einer Konzentration entsprechend 0,1 bis 20 Gew.-% Aluminiumoxid, bezogen auf die Summe der Gewichte von Aluminiumoxid plus Siliciumdioxid, verwendet.

Als Zinkverbindung kann beispielsweise Natriumzinkat und/ oder eine der sonstigen vorstehend genannten Zinkverbindungen verwendet werden in einer Konzentration entsprechend 0,1 bis 50 Gew.-% Zinkoxid, bezogen auf das im Reaktionsgemisch vorhandene bzw. entstehende Aluminiumsilikat.

Als Stickstoffverbindungen kommen insbesondere Alkylammoniumverbindungen, primäre, sekundäre oder tertiäre Monoamine, lineare oder cyclische Diamine und/oder hydroxysubstituierte Verbindungen vom Typus der Alkanolamine in Frage, beispielsweise seien hier Tetrapropylammoniumhalogenide oder -hydroxid, Tetrabutylammoniumhalogenide oder -hydroxid, Hexamethylendiamin, Ethanolamin, Propylamin, Butylamin und/oder Cholin genannt, die in einer Menge von 5 bis 100 Gew.-% (bezogen auf vorhandenes oder sich bildendes Aluminiumsilikat) eingesetzt werden. In einem alkali- und druckbeständigen Reaktionsgefäß wird dann bei 100 bis 150°C im Verlaufe von 10 bis 25 Tagen das amorphe Siliciumdioxid in weitgehend kristallines zinkhaltiges Aluminiumsilikat überführt.

Als Ausgangsmaterial kann auch ein überwiegend amorphes Aluminiumsilikat verwendet werden, das bereits einen Aluminiumoxidgehalt im Bereich von 0,1 bis 20 Gew.-% aufweist. Dann kann selbstverständlich auf den in der vorstehenden beispielhaften technischen Ausführungsform angegebenen Zusatz einer oder mehrerer Aluminiumverbindungen verzichtet werden, ohne daß sich das Verfahren sonst wesentlich ändert.

Nach Beendigung der Kristallisation, deren Fortgang beispielsweise durch eine Röntgenbeugungsanalyse verfolgt werden kann, wird die evtl. vorhandene wäßrige

Phase mechanisch abgetrennt. Diese kann, gegebenenfalls nach Ergänzung der verbrauchten Bestandteile, einer Wiederverwendung zugeführt werden. Durch Waschen mit Wasser entfernt man überschüssiges Alkali und trocknet das Reaktionsprodukt. Anschließend wird dieses bei 400 bis 600°C calciniert, dann solange mit einer wäßrigen Ammoniumsalzlösung behandelt, bis der überwiegende Teil der im Reaktionsprodukt vorhandenen austauschbaren Natriumionen durch Ammoniumionen ersetzt ist. Dann wird nochmals getrocknet und calciniert und so ein Katalysator erhalten, der beispielsweise für die Umwandlung von Methanol und/oder Dimethylether in Kohlenwasserstoffe geeignet ist.

Selbstverständlich können die erfindungsgemäß hergestellten Katalysatoren vor oder nach dem Ionenaustausch $Na^+$ gegen $NH_4^+$ mit Binde- und/oder Verfestigungsmitteln versetzt und nach den üblicherweise angewendeten Techniken zu Katalysatoren für Fest-, Flugstaub-, Wirbel- oder Wanderbettanlagen verarbeitet werden.

Im Falle der erfindungsgemäßen Umwandlung geformter Ausgangsmaterialien in zinkhaltiges, kristallines Aluminiumsilikat erübrigt sich selbstverständlich ein Zusatz von Binde- oder Verfestigungsmitteln, da die Katalysatoren dann in der Form des Ausgangsmaterials vorliegen.

In den erfindungsgemäßen Katalysatoren ist das darin enthaltene Zink im allgemeinen mit den üblichen Ionenaustauschtechniken nicht restlos entfernbar, was auf einen besonders festen Einschluß des Zinks im Silikatgitter schließen läßt.

Die vorliegende Erfindung betrifft weiterhin die Verwendung von zinkhaltigen Aluminiumsilikat-Katalysatoren, die erhältlich sind, indem man amorphes Aluminiumsilikat mit einem Gehalt an Aluminiumoxid von 0,1 bis 20 Gew.-% oder Gemische, aus denen ein solches Aluminiumoxid erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternären Ammoniumverbindungen, sowie in wäßrigen Alkalilösungen löslichen oder teilweise löslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert, zur Umwandlung von niederen Alkoholen und/oder Ethern in Kohlenwasserstoffe.

Die folgenden Beispiele erläutern die vorliegende Erfindung ohne sie in irgendeiner Weise einzuschränken.

Beispiele

Beispiel 1

Dieses Beispiel betrifft die Herstellung eines zinkhaltigen Aluminiumsilikats aus einem handelsüblichen amorphen Aluminiumsilikat.

1000 g eines in Form von Kugeln mit einem Durchmesser von 5 mm vorliegenden amorphen Aluminiumsilikats, das nach Spezifikation einen $Al_2O_3$-Gehalt von unter 5 Gew.-% aufweist und unter der Bezeichnung KA von der Südchemie AG, München, Deutschland, bezogen werden kann, wurden 6 Stunden bei 900°C geglüht und anschließend durch Evakuieren entgast.

Das so vorbehandelte Material wurde mit einer Mischung, bestehend aus 80 g Natriumhydroxid, 81 g Zinkoxid, 266 g Tetrapropylammoniumbromid und 1400 g Wasser, übergossen und während eines Zeitraumes von 20 Tagen einer Temperatur von 100°C ausgesetzt.

Anschließend wurde die Mutterlauge durch Abdekantieren entfernt und das Reaktionsprodukt, das noch die ursprüngliche Kugelform aufwies, bis zur neutralen Reaktion mit Wasser gewaschen.

Die Trocknung dieses Produkts bei 120°C und 2-stündiges Calcinieren bei 550°C führt zu einem zinkhaltigen und kristallinen Aluminiumsilikat mit der Zusammensetzung (als Oxidgemisch berechnet):

| | |
|---|---|
| $SiO_2$ | 92,20 % |
| $Al_2O_3$ | 1,51 % |
| $Na_2O$ | 1,22 % |
| ZnO | 5,07 % |

Zur Entfernung der austauschbaren Natriumionen wurde das Produkt in einer beheizten Chromatographie-Säule bei einer Temperatur von 100°C kontinuierlich mit insgesamt 10 l einer 2 n-Ammoniumchloridlösung behandelt, danach getrocknet und bei einer Temperatur von 500°C bis zum Ende der Entwicklung flüchtiger Anteile calciniert.

Es resultierte die Wasserstoff-Form eines zinkhaltigen kristallinen Aluminiumsilikats mit besonderen katalytischen Eigenschaften, wie aus den nachfolgenden Beispielen hervorgeht.

Beispiel 2

Dieses Beispiel betrifft die Verwendung des gemäß Beispiel 1 hergestellten zinkhaltigen Aluminiumsilikats als Katalysator für die Umwandlung von Methanol in Kohlenwasserstoffe:

314 ml des nach Beispiel 1 hergestellten Katalysators wurden in einen Rohrreaktor gefüllt und bei einer Versuchstemperatur von 360°C, einem Druck von 10 bar und einer Kontaktbelastung von 444 g/l . h mit gasförmigem Methanol beaufschlagt. Der Reaktionsdruck wurde durch einen Stickstoffzusatz zum Einsatz-Methanol aufrechterhalten.

Die Versuchsergebnisse sind in Tabelle 1 dargestellt. Es ist zu erkennen, daß sich der Katalysator, selbst wenn er in konventioneller Weise bei Temperaturen von ca. 500°C regeneriert wird, durch eine außergewöhnlich hohe Standfestigkeit auszeichnet (Dauer des 2. und 3. Reaktionszyklus 672 bzw. 643 Stunden).

Tabelle 1

| Reaktionszyklus Nr. | 1 | | | 2 | | | 3 | |
|---|---|---|---|---|---|---|---|---|
| | Reaktion bei 360°C | | Regeneration bei 500°C | Reaktion bei 360°C | | Regeneration bei 500°C | Reaktion bei 360°C | |
| Zyklusdauer h | 564 | | | 672 | | | 643 | |
| Methanolumsatz, Gew.% total | Anfang 99,0 | Ende 97,9 | analog zu c) | Anfang 99,9 | Ende 98,6 | analog zu c) | Anfang 99,9 | Ende 98,7 |
| Methanolumsatz zu Gew.% Kohlenwasserstoffen a) | 99,9 | 95,9 | | 98,7 | 95,5 | | 98,2 | 92,5 |
| Methanolumsatz zu Gew.% Dimethylether b) | 0,0 | 2,0 | | 1,2 | 3,1 | | 1,7 | 6,2 |

| | 4 | | | 5 | |
|---|---|---|---|---|---|
| Regeneration bei 360°C | Reaktion bei 360°C | | Regeneration bei 360°C | Reaktion bei 360°C | |
| | 821 | | | 720 | |
| mit Luft und $H_2O$ d) | Anfang 99,9 | Ende 98,2 | mit Luft und $H_2O$ d) | Anfang 99,9 | Ende 98,6 |
| | 98,2 | 94,0 | | 98,1 | 94,4 |
| | 1,7 | 3,4 | | 1,0 | 4,2 |

Erklärungen zu Tabelle 1

a) entspricht der Gesamtausbeute an Kohlenwasserstoffen plus Reaktionswasser

b) entspricht der Ausbeute an Dimethylether plus Reaktionswasser

c) Regeneration nach Ind. Eng. Chem. Process Des. Dev., Vol. 17, No 3, Seite 341 (1978)

d) Regenerierbedingungen: 2000 g $H_2O$/l Katalysator und Stunde (verdampft und auf 360°C vorgeheizt) zusätzlich Luftzugabe entsprechend einer Anfangskonzentration von 1 % $O_2$, bezogen auf das den Katalysator durchströmende Dampf-Luft-Gemisch bis zum Abklingen der $CO_2$-Entwicklung, dann kontinuierliche Steigerung des Sauerstoffgehaltes auf 10 Vol.-% durch Erhöhung der Luftzugabe bis zum Absinken des Sauerstoff-Umsatzes auf 0 %, Regenerationsdruck 10 bar.

Die Tabelle 1 zeigt weiterhin, daß der Katalysator hinsichtlich seiner Standfestigkeit, gekennzeichnet durch die Dauer eines Reaktionszyklus, noch bessere Ergebnisse zeigt, wenn er bei 360°C mit einem Wasserdampf-Luft-Gemisch regeneriert wird. So sind die Zyklen 4 und 5 mit 821 bzw. 720 Stunden Dauer wesentlich länger als die vorangegangenen. Der Katalysator ist somit für einen technischen Einsatz in jeder Hinsicht geeignet, und er besitzt überlegene Eigenschaften hinsichtlich Standfestigkeit und Regenerierbarkeit.

Beispiel 3

Dieses Beispiel betrifft die katalytische Umwandlung eines Dimethylether-Wasser-Gemisches in Kohlenwasserstoffe an einem nach Beispiel 1 hergestellten Katalysator und unter sogenannter Kreisgasfahrweise (Rückführung eines Teils der gasförmigen Reaktionsprodukte in den Reaktor).

Eine Mischung aus 83 Gew.-% Methanol und 17 Gew.-% Wasser wurde bei 300°C verdampft und über einen extrudierten Dehydratisierungskatalysator auf Basis eines dekationisierten amorphen Aluminiumsilikats geleitet. Die Kontaktbelastung betrug 10 g Methanol/Wasser-Mischung pro Gramm Katalysator und Stunde. Es wurde bei einem Druck von 10 bar gearbeitet. Eine Analyse des den Reaktor verlassenden Gasgemisches zeigte das Vorliegen von Dimethylether, Wasser und einen Rest an nicht-umgesetztem Methanol an.

Tabelle 2

| Reaktionszyklus Nr. | 1 | | 2 | | 3 | | 4 | |
|---|---|---|---|---|---|---|---|---|
| | Reaktion | Regeneration | Reaktion | Regeneration | Reaktion | Regeneration | Reaktion | Regeneration |
| durchschnittl. Temp. im Reaktor (°C) | 370 | 370 | 370 | 370 | 370 | 370 | 370 | 370 |
| Zyklusdauer a) (h) | 300 | b) | 300 | b) | 300 | b) | 300 | b) |
| Ausbeute an Kohlenwasserstoffen plus Reaktionswasser Anfangswert, Endwert (Gew.-%) | 100-99,8 | | 100-100 | | 100-100 | | 100-99,7 | |

| Reaktionszyklus Nr. | 5 | | 6 c) |
|---|---|---|---|
| | Reaktion | Regeneration | Reaktion |
| durchschnittl. Temp. im Reaktor (°C) | 370 | 370 | 370 |
| Zyklusdauer a) (h) | 300 | b) | 300 |
| Ausbeute an Kohlenwasserstoffen plus Reaktionswasser Anfangswert, Endwert (Gew.-%) | 100-99,8 | | 100-100 |

Patentansprüche

1) Zinkhaltiger Aluminiumsilikat-Katalysator, dadurch gekennzeichnet, daß er erhältlich ist, indem man amorphes Aluminiumsilikat mit einem Gehalt an Aluminiumoxid von 0,1 bis 20 Gew.-% oder Gemische, aus denen ein solches Aluminiumsilikat erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternären Ammoniumverbindungen, sowie mit in wäßrigen Alkalilösungen löslichen oder teilweise löslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert.

2) Verfahren zur Herstellung von zinkhaltigen Aluminiumsilikat-Katalysatoren, dadurch gekennzeichnet, daß man amorphes Aluminiumsilikat mit einem Gehalt an Aluminiumoxid von 0,1 bis 20 Gew.-% oder Gemische, aus denen ein solches Aluminiumsilikat erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternären Ammoniumverbindungen, sowie mit in wäßrigen Alkalilösungen löslichen oder teilsweise löslichen Verbindungen des Zinks, in einem wäßrigen Milieu, bei einem pH-Wert von größer als 8, Tempe-

raturen von 50 bis 250°C aussetzt, anschließend auswäscht, trocknet und bei 300 bis 600°C calciniert.

3) Verfahren nach Anspruch 2, dadurch gekennzeichnet, daß man amorphes Aluminiumsilikat mit einem Gehalt an Aluminiumoxid von 0,1 bis 20 Gew.-% einsetzt.

4) Verfahren nach Anspruch 2, dadurch gekennzeichnet, daß man ein Gemisch aus amorphem Siliciumdioxid mit einem Gehalt an Siliciumdioxid von über 90 Gew.-% und löslichen, als Quelle für Aluminiumoxid und/oder Aluminiumhydroxid geeigneten Verbindungen des Aluminiums entsprechend einem Gehalt von 0,1 bis 20 Gew.-% Aluminiumoxid, bezogen auf die Summe der Gewichte von Siliciumdioxid und Aluminiumoxid, einsetzt.

5) Verfahren nach Ansprüchen 2 bis 4, dadurch gekennzeichnet, daß man Formkörper aus amorphem Aluminiumsilikat bzw. amorphem Siliciumdioxid einsetzt.

6) Verfahren nach Anspruch 5, dadurch gekennzeichnet, daß die Formkörper einen minimalen Durchmesser von über 1 mm und einen maximalen Durchmesser von unter 20 mm aufweisen.

7) Verfahren nach Ansprüchen 2 bis 6, dadurch gekennzeichnet, daß man die Verbindungen des Zinks in einer Menge entsprechend einem Gehalt von 0,5 bis 10 Gew.-% Zinkoxid, bezogen auf das vorhandene oder entstehende Aluminiumsilikat, einsetzt.

8) Verfahren nach Ansprüchen 2 bis 7, dadurch gekennzeichnet, daß man als Zinkverbindung Zinkoxid, Zinkhydroxid, Zinkate, Zinksalze und/oder komplexe Zinkverbindungen einsetzt.

9) Verfahren nach Ansprüchen 2 bis 8, dadurch gekennzeichnet, daß man das Reaktionsprodukt einem Ionenaustausch mit Ammonium- oder Wasserstoffionen unterzieht und nochmals calciniert.

10) Verwendung von zinkhaltigen Aluminiumsilikat-Katalysatoren, die erhältlich sind, indem man amorphes Aluminiumsilikat mit einem Gehalt an Aluminiumoxid von 0,1 bis 20 Gew.-% oder Gemische, aus denen ein solches Aluminiumoxid erhältlich ist, mit aliphatischen $C_2$- bis $C_{24}$-Aminen und/oder cycloaliphatischen $C_4$- bis $C_{24}$-Aminen und/oder Ammoniumsalzen dieser Amine und/oder den aus diesen Aminen durch Quarternierung erhältlichen quarternären Ammoniumverbindungen, sowie mit in wäßrigen Alkalilösungen löslichen oder teilweise löslichen Verbindungen des Zinks, in einem wäßrigen Milieu, bei einem pH-Wert von größer als 8, Temperaturen von 50 bis 250°C aussetzt, anschließend auswäscht, trocknet und bei 300 bis 600°C calciniert, zur Umwandlung von niederen Alkoholen und/oder Ethern in Kohlenwasserstoffe.